# EUROPEAN PATENT APPLICATION

(11) **EP 4 129 226 A1**
(43) Date of publication of application: **08.02.2023**
(21) Application number: 20932776.6
(22) Date of filing: 24.04.2020
(51) Int. Cl.: A61B 34/30

(54) **SURGICAL SYSTEM**

(71) Applicant: Riverfield Inc., Tokyo 107-0052 (JP)
(72) Inventor: YAMAMOTO, Nobuaki, Tokyo 160-0017 (JP)
(74) Representative: adares Patent- und Rechtsanwälte Reininger & Partner GmbB
(86) International application number: PCT/JP2020/017692
(87) International publication number: WO 2021/214977

(57) **Abstract**

An aspect of the surgical system, which is capable of optimally controlling the initial position of a treatment tool based on multifaceted information regarding surgery, includes: a gripping robot that holds a treatment tool; and an initial setting unit that sets an initial state including an initial position of the gripping robot. The initial setting unit includes: a storage unit that stores relevance information pertaining to a relationship between condition information regarding surgery and the initial position of the gripping robot; a position specifying unit that sets, based on the condition information input and the relevance information input from the storage unit, the initial position of the gripping robot; and a drive control unit that generates, based on a signal input from the position specifying unit, a control signal for moving a position of the gripping robot to a certain position and outputs the control signal to the gripping robot.

## Description

### [Technical Field]

The present invention relates to a surgical system using a gripping robot that holds a treatment tool such as an endoscope.

### [Background Art]

In surgery using an endoscope such as a laparoscope, the abdomen of a patient is provided with two or more holes, through which an endoscope, forceps, etc. are inserted, and the surgeon operates the surgical tool such as forceps while observing an image captured by the endoscope. Patent Document 1 discloses a surgical system using a gripping robot that holds an endoscope. This system includes a surgical treatment device that allows a surgeon to accurately determine the positions of an endoscopic camera and other surgical tools in a patient without removing his/her hands from the tools.

This surgical treatment device includes a robotic manipulator having at least one controllable degree of freedom, a means that controls the operation of the manipulator, a device holding means for attaching a first surgical device to the manipulator, and a surgeon input means that is mounted on another surgical device and allows the surgeon to specify an operation required for the first surgical device.

### [Prior Art Documents]

### [Patent Documents]

[Patent Document 1] JP2575586B

### [Summary of the Invention]

### [Problems to be solved by the Invention]

Thus, the surgical system using a gripping robot that holds an endoscope can accurately control the position of the endoscope in accordance with surgical information, and also from the viewpoint of supporting the surgeon and reducing the burden on the patient, the role of the surgical system is becoming more and more important. On the other hand, the position of the endoscope can be accurately controlled by using the gripping robot, but the endoscope may not necessarily be controlled to a position that is preferred to the surgeon.

An object of the present invention is to provide a surgical system capable of optimally controlling the initial position of a treatment tool based on multifaceted information regarding surgery.

### [Means for solving the Problems]

To solve the above problem, an aspect of the present invention provides a surgical system including: a gripping robot that holds a treatment tool; and an initial setting unit that sets an initial state including an initial position of the gripping robot. The initial setting unit includes: a storage unit that stores relevance information pertaining to the relationship between condition information regarding surgery and the initial position of the gripping robot; a position specifying unit that sets, based on the condition information input and the relevance information input from the storage unit, the initial position of the gripping robot; and a drive control unit that generates, based on a signal input from the position specifying unit, a control signal for moving the position of the gripping robot to a certain position and outputs the control signal to the gripping robot.

According to such a configuration, the initial state of the gripping robot is set based on the relevance information pertaining to the relationship between the condition information regarding surgery and the initial position of the gripping robot, and the treatment tool held by the gripping robot can therefore be set to an optimum initial position based on the information regarding the surgery.

In the above surgical system, preferably, the position specifying unit generates new relevance information based on a surgery performed immediately before by a surgeon and stores the new relevance information in the storage unit in association with the surgeon. This allows the initial state of the gripping robot in the next surgery to be set based on the information on the initial state of the gripping robot in the surgery performed immediately before by the surgeon.

In the above surgical system, the condition information may include one or more selected from the group consisting of surgeon information, surgical procedure information, patient information, target organ information, treatment tool product information, and adapter information of an adapter that is connected to the gripping robot and directly holds the treatment tool. This allows the initial state of the gripping robot to be set based on information from various viewpoints regarding the surgery.

In the above surgical system, the surgical procedure information may include information regarding the port position of a trocar. This allows the initial state of the gripping robot to be set so that the treatment tool can be aligned corresponding to the port position of the trocar used in the surgery.

In the above surgical system, the patient information may include body shape, age, and gender. This allows the initial state of the gripping robot to be set based on the information specified by the patient's body shape, age, and gender.

In the above surgical system, the treatment tool product information may include information regarding the shape of the treatment tool. This allows the initial state of the gripping robot to be set based on the information regarding the shape of the treatment tool.

In the above surgical system, the condition information may include at least one of information input from an input device, information on an electronic medical record, and information that is preliminarily stored in the storage unit. This allows the initial state of the gripping robot to be set based on various condition information items.

In the above surgical system, the storage unit may also store the relationship between the condition information and another setting in an operating room as the relevance information, and the initial setting unit may have a condition setting unit that specifies another setting condition in the operating room based on the condition information input and the relevance information input from the storage unit. This allows the other setting condition in the operating room to be set based on the condition information regarding the surgery in addition to setting the initial state of the gripping robot.

### [Effect of the Invention]

According to the present invention, it is possible to provide a surgical system capable of optimally controlling the initial position of a treatment tool based on multifaceted information regarding surgery.

### [Brief Description of Drawings]

FIG. 1 is a block diagram exemplifying the configuration of a surgical system according to an embodiment of the present invention.
FIG. 2 is a schematic diagram exemplifying an endoscope as an example of a treatment tool.
FIG. 3 is a schematic diagram illustrating an example of a gripping robot.
FIG. 4 is a schematic diagram illustrating a usage example of the surgical system according to the present embodiment.
FIG. 5 is a diagram for describing an affected area for which surgery using an endoscope is performed.
FIG. 6 is a diagram for describing the insertion direction of an endoscope when operating a lung.
FIG. 7 is a diagram for describing the insertion direction of an endoscope when operating a liver.
FIG. 8(a) is a diagram for describing the insertion direction of an endoscope when operating a kidney with a transperitoneal approach, and FIG. 8(b) is a diagram for describing the insertion direction of an endoscope when operating a kidney with a retroperitoneal approach.

### [Embodiments for Carrying out the Invention]

Hereinafter, one or more embodiments of the present invention will be described with reference to the drawings. In the following description, the same members are denoted by the same reference numerals and the description of members once explained may be omitted.

### (Overall Configuration of Surgical System)

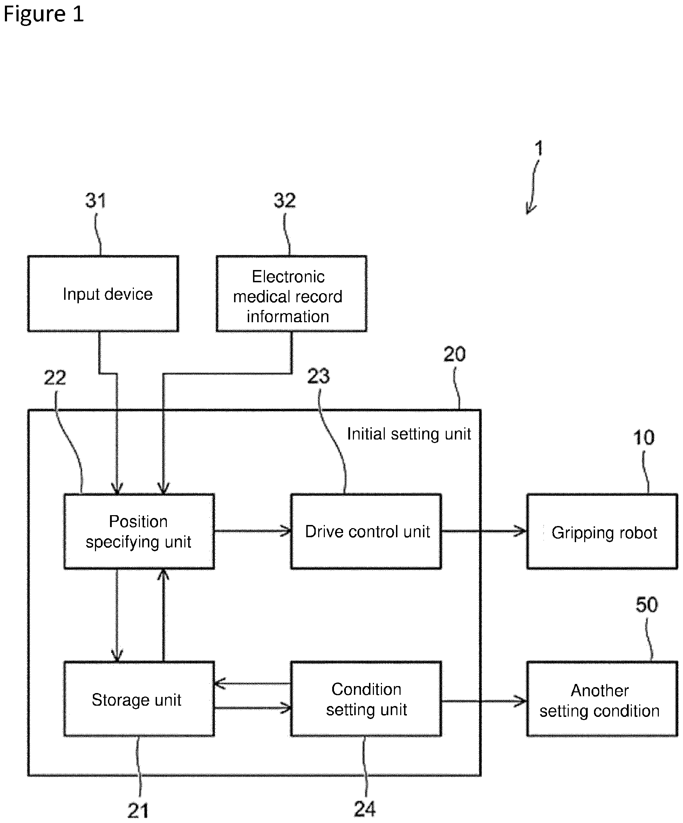

FIG. 1 is a block diagram exemplifying the configuration of a surgical system according to an embodiment of the present invention.

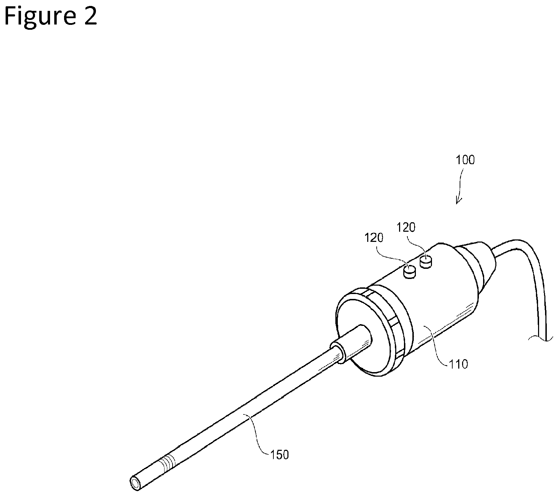

FIG. 2 is a schematic diagram exemplifying an endoscope as an example of a treatment tool.

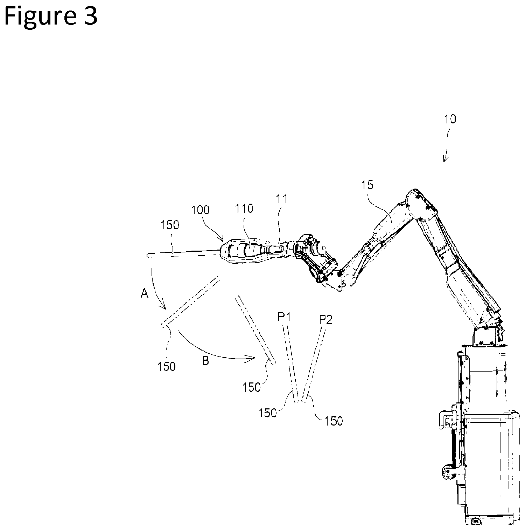

FIG. 3 is a schematic diagram illustrating an example of a gripping robot.

A surgical system 1 according to the present embodiment includes a gripping robot 10 that holds a treatment tool, and an initial setting unit 20 that sets an initial state including the initial position of the gripping robot 10. In the present embodiment, a case in which an endoscope 100 is used as the treatment tool will be described as an example.

As illustrated in FIG. 2, the endoscope 100 has a configuration in which a given lens barrel 150 is attached to a head portion 110. The lens barrel 150 is detachably attached to the head portion 110, so that the lens barrel 150 suitable for surgery can be replaced and used. The head portion 110 is provided with operation units 120. The operation of the endoscope 100 can be performed by operating the operation units 120. For example, the operation units 120 are used to adjust the zoom in imaging with the endoscope 100 and adjust the imaging range. In the type in which the tip of the lens barrel 150 is bent, the operation units 120 can be operated to bend the tip of the lens barrel 150 in a desired direction. When the endoscope 100 is used in laparoscopic surgery or the like, the lens barrel 150 of the endoscope 100 is inserted into the abdomen of a patient, and the image or video in the abdomen is captured and displayed on a monitor. The surgeon operates forcipes and the like while observing the image or video of an affected area displayed on the monitor to perform the surgery.

As illustrated in FIG. 3, the endoscope 100 is held by the gripping robot 10 and its position is accurately controlled. The gripping robot 10 has an arm 15 and an adapter 11 provided at the tip of the arm 15 for holding the endoscope 100. The position of the endoscope 100 is controlled by driving the arm 15 of the gripping robot 10. The arm 15 is a multi-joint type, and the position and angle of the endoscope 100 can be freely set by the motion of the arm 15.

### (Initial Setting Unit of Surgical System)

As illustrated in FIG. 1, the initial setting unit 20 of the surgical system 1 includes a storage unit 21, a position specifying unit 22, and a drive control unit 23. The storage unit 21 stores relevance information pertaining to the relationship between condition information regarding surgery and the initial position of the gripping robot 10.

Here, the initial position of the gripping robot 10 is related to the initial position of the endoscope 100 held by the gripping robot 10 when performing the surgery (the position set at the stage of starting the surgery). That is, the endoscope 100 is held on the arm 15 of the gripping robot 10 thereby to determine the positional relationship between the gripping robot 10 and the endoscope 100. This allows the initial position of the tip of the endoscope 100 to be determined based on the initial position of the gripping robot 10.

The condition information regarding the surgery includes one or more selected from the group consisting of surgeon information, surgical procedure information, patient information, target organ information, treatment tool product information, and adapter information of an adapter that is connected to the gripping robot 10 and directly holds a treatment tool (e.g., the endoscope 100).

The surgeon information includes information on each surgeon, such as the surgeon's height, dominant arm, visual acuity, color vision, and intraoperative position (standing position, whether to sit on a chair). The surgical procedure information includes information regarding the type of a treatment tool, the arrangement of treatment tools, and the port position of a trocar depending on the content of surgery. The patient information includes information on the patient's body shape (height, weight), age, and gender. The target organ information includes information such as the name of an organ as the target of surgery and the positional relationship between the organ and the main blood vessel. The treatment tool product information includes information regarding the shape of a treatment tool. The adapter information includes information regarding a treatment tool attachment reference position of the adapter 11 provided in the gripping robot 10 which holds a treatment tool.

These condition information items may be input from an input device 31 such as a keyboard or a scanner or may also be taken in from information such as electronic medical record information 32. Additionally or alternatively, the condition information may be information that is preliminarily stored in the storage unit 21.

The position specifying unit 22 sets the initial state of the gripping robot 10 based on the condition information input and the relevance information input from the storage unit 21. The initial state of the gripping robot 10 includes the initial position of the gripping robot 10. Examples of the initial state other than the initial position include the angles of the arm 15 and adapter 11 when determining the initial position of the gripping robot 10. Details of the position specifying unit 22 will be described later.

The drive control unit 23 generates a control signal for moving the position of the gripping robot 10 to a certain position based on the signal input from the position specifying unit 22 and outputs the control signal to the gripping robot 10.

In the surgical system 1 having such a configuration, the initial position of the gripping robot 10 is set based on the relevance information pertaining to the relationship between the condition information regarding the surgery and the initial position of the gripping robot 10. For example, the position specifying unit 22 uses the surgical procedure information, the organ information, and the surgeon information to specify a position on the body surface of a patient from which the endoscope 100 is inserted and a direction in which the endoscope 100 is inserted, and the drive control unit 23 controls the gripping robot 10 so that the endoscope 100 is set to the specified position. This allows the endoscope 100 held by the gripping robot 10 to be set to an optimum initial position.

Conventionally, when the holding robot 10 holds the endoscope 100 and controls its position, the initial position of the endoscope 100 is not determined or is set to a fixed initial position, and the endoscope 100 is controlled from that position. In the surgical system 1 according to the present embodiment, the position specifying unit 22 specifies an optimum initial position of the endoscope 100 by using the condition information regarding the surgery, and the gripping robot 10 is controlled so that the endoscope 100 is set to that position. This allows the initial position of the endoscope 100 to be set, for example, in accordance with the surgical procedure, the body shape of the patient, and the preference of the surgeon.

Even in the same surgery, it may be better to change the position of the arm 15 of the gripping robot 10 depending on the height, dominant arm, standing position, etc. of the surgeon. With consideration for such information, the gripping robot 10 can be disposed at a position at which the surgeon can easily perform the surgery, and the initial position of the endoscope 100 can be set.

### (Position Specifying Unit)

To specify the initial position of the endoscope 100, the position specifying unit 22 sets the initial state of the gripping robot 10 based on the condition information input and the relevance information input from the storage unit 21. When the gripping robot 10 is fixed to a surgical bed, the position control of the gripping robot 10 is performed with reference to a specific position of the surgical bed or the gripping robot 10. On the other hand, if the gripping robot 10 is not fixed to a surgical bed, the origin of the position control of the gripping robot 10 is set when the relative positional relationship between the surgical bed and the gripping robot 10 is determined. When each of the gripping robot 10 and the surgical bed has absolute position information, the position control of the gripping robot 10 is performed in accordance with the absolute position reference. The position specifying unit 22 specifies the initial state of the gripping robot 10 by using any of the above position references.

The position specifying unit 22 specifies the initial state of the gripping robot 10 by using the condition information input with the input device 31 and/or the condition information read from the electronic medical record information 32. By specifying the initial state of the gripping robot 10, it is possible to specify the initial position of the endoscope 100 held by the gripping robot 10.

Additionally or alternatively, the position specifying unit 22 may use the product information (such as the shape and type) of the endoscope 100 and/or the information on the adapter for the endoscope 100 in the gripping robot 10 to determine the positional relationship between the tip of the gripping robot 10 and the tip of the endoscope 100 and specify the initial position of the endoscope 100. The insertion position of the endoscope 100 in the patient is determined by the patient information in relation to the origin of the gripping robot 10.

The position specifying unit 22 may specify the initial position of the endoscope 100 by using the information regarding the port position of a trocar included in the surgical procedure information. That is, the port position of the trocar is set in accordance with the surgical procedure, and the initial position of the endoscope 100 may be specified so that its tip is directed toward the port position calculated based on the port position of the trocar to be a reference and the patient information (patient's body shape).

Additionally or alternatively, the position specifying unit 22 may generate new relevance information based on the surgery performed immediately before by the surgeon and store the new relevance information in the storage unit 21 in association with the surgeon. For example, even when the endoscope 100 is set to the initial position specified by the position specifying unit 22, the surgeon may start inserting the endoscope 100 at that position or may otherwise correct the position to some extent before inserting the endoscope 100. When the position is corrected, the position specifying unit 22 stores the corrected position in the storage unit 21 as new relevance information in association with the current condition information. This allows the initial position of the gripping robot 10 in the next surgery to be set based on the information on the initial position of the gripping robot 10 in the surgery performed immediately before by the surgeon. The relevance information thus obtained can also be used as teacher data for machine learning of the relationship between the condition information and the position information.

Laparoscopic surgery is performed by sending gas into the abdomen of a patient and inflating the abdomen. Accordingly, the position from which the endoscope 100 should be inserted (the position of the trocar) may often be different from the originally planned position or orientation. Therefore, after the endoscope 100 is set to the initial position specified by the position specifying unit 22, the surgeon guides the endoscope 100 to an accurate position. In this operation, by accumulating the data on a deviation between the initial position and the actual position, the position specifying unit 22 can improve the accuracy when specifying the initial position of the endoscope 100 in the next surgery.

The position specifying unit 22 may obtain the deviation between the initial position of the endoscope 100 and the actual insertion position by analyzing the image or video taken in by the endoscope 100. For example, after the endoscope 100 is set to the initial position, the image or video is acquired by the endoscope 100. The position of the trocar is obtained from this image or video by image recognition, and the deviation between the endoscope 100 and the position of the trocar is calculated. The position specifying unit 22 may send a drive signal to the drive control unit 23 so as to correct the deviation obtained by that calculation, and the gripping robot 10 may be driven to align the position of the endoscope 100 with the actual position of the trocar.

The storage unit 21 may also store the relationship between the condition information and another setting in the operating room as the relevance information, and the initial setting unit 20 may have a condition setting unit 24 that specifies another setting condition in the operating room based on the condition information input and the relevance information input from the storage unit 21. This allows the initial position of the gripping robot 10 to be set based on the other setting condition in the operating room in addition to the information regarding the operating room.

In the surgical system 1 according to the present embodiment, the initial setting unit 20 may include the condition setting unit 24. The condition setting unit 24 specifies another setting condition 50 (see FIG. 1) in the operating room based on the condition information input and the relevance information input from the storage unit 21. Here, specific examples of the other setting condition in the operating room include the temperature, humidity, and lighting condition of the operating room. The storage unit 21 also stores the relationship between the condition information and the other setting in the operating room as the relevance information, and the condition setting unit 24 specifies the other setting condition in the operating room based on the relevance information stored in the storage unit 21 and the condition information input. This allows the other setting condition in the operating room to be set based on the condition information regarding the surgery in addition to specifying the initial state of the gripping robot 10, and the surgical environment can be optimized. Other specific examples of the other setting condition in the operating room include the setting of a treatment tool. More specifically, when the treatment tool is the endoscope 100, such specific examples include the optical settings (such as illumination and focal position) and operational settings (such as heater temperature) of the endoscope 100.

### (Usage Example)

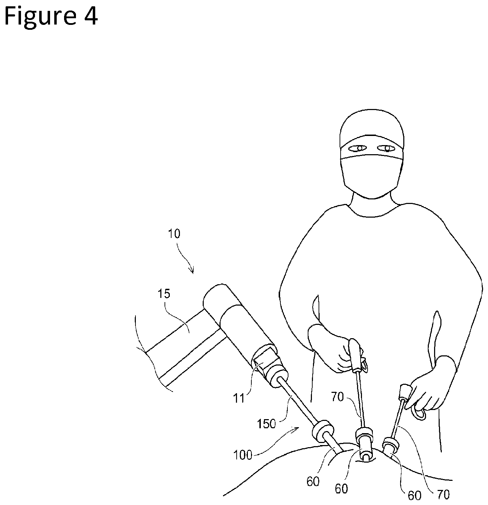

FIG. 4 is a schematic diagram illustrating a usage example of the surgical system according to the present embodiment.

FIG. 4 illustrates a usage example of the surgical system 1 during laparoscopic surgery. In laparoscopic surgery, trocars 60 are inserted by making two or more small holes in the abdomen of a patient, and the endoscope 100 and forcipes 70 are inserted through the trocars 60 for the surgery. In the example illustrated in FIG. 4, the endoscope 100 is held by the arm 15 of the gripping robot 10, and the lens barrel 150 of the endoscope 100 is inserted into the body of the patient from a trocar 60. The forcipes 70 operated by the surgeon are inserted from the other trocars 60.

When starting the laparoscopic surgery, the initial state of the gripping robot 10 is specified by the position specifying unit 22 of the initial setting unit 20, and the gripping robot 10 is controlled by the drive control unit 23 so as to come to the initial state. The initial state of the gripping robot 10 is controlled, and the endoscope 100 held by the gripping robot 10 is thereby set to the initial position.

For example, as illustrated in FIG. 3, after the endoscope 100 is attached to the adapter of the gripping robot 10, the arm 15 of the gripping robot 10 is driven to change the position and orientation of the endoscope 100 as illustrated by an arrow A to an arrow B in the figure. When the initial position of the endoscope 100 specified based on certain condition information is a position P1 illustrated in the figure, the position specifying unit 22 specifies the initial state of the gripping robot 10 so that the endoscope 100 is located at the position P1. By driving the arm 15 of the gripping robot 10 to the specified initial state, the endoscope 100 is set to the position P1.

On the other hand, depending on the condition information, an initial position different from the position P1 may be specified. When a position P2 illustrated in the figure is specified, the gripping robot 10 is controlled to align the initial position of the endoscope 100 with the position P2. Thus, the initial state of the gripping robot 10 based on the optimum initial position of the endoscope 100 is specified by the condition information, and the endoscope 100 is set to the optimum initial position by controlling the gripping robot 10 to that initial state.

The surgeon operates the forcipes 70 to perform the surgery while referring to the image or video of the surgical site captured by the endoscope 100 on the monitor. The position of the endoscope 100 may be controlled by the gripping robot 10 as the surgery progresses.

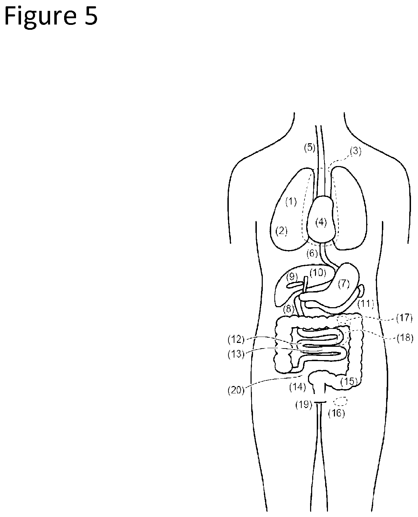

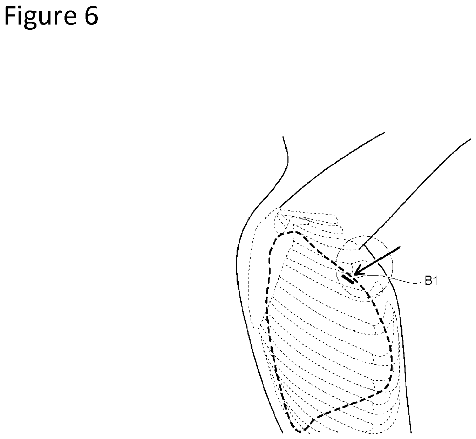

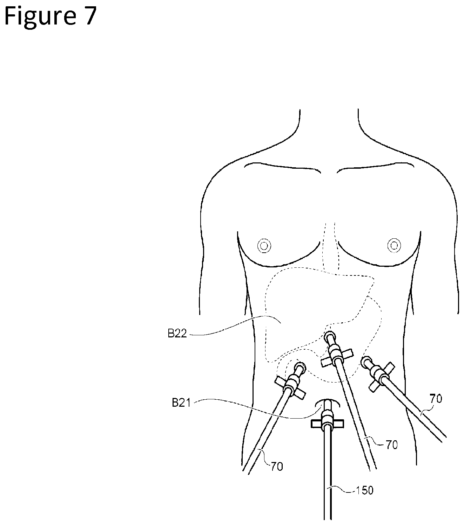

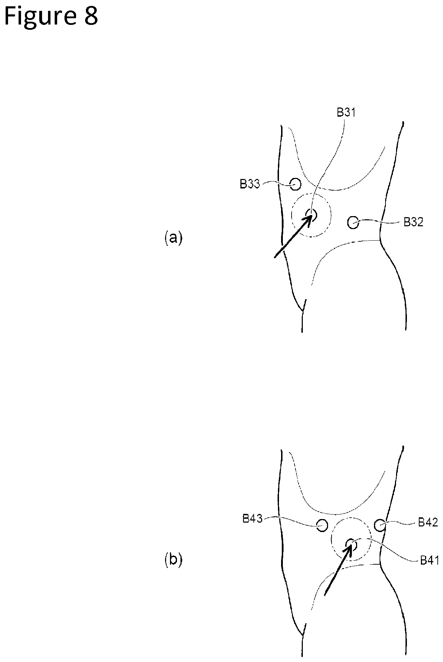

FIG. 5 is a diagram for describing an affected area for which surgery using an endoscope is performed. FIG. 6 is a diagram for describing the insertion direction of an endoscope when operating a lung. FIG. 7 is a diagram for describing the insertion direction of an endoscope when operating a liver. FIG. 8(a) is a diagram for describing the insertion direction of an endoscope when operating a kidney with a transperitoneal approach. FIG. 8(b) is a diagram for describing the insertion direction of an endoscope when operating a kidney with a retroperitoneal approach.

By using the endoscope 100, it is possible to perform surgery on various sites of a body as the targets. The scope of application is broadly classified into thoracoscopic surgery and laparoscopic surgery. As illustrated in FIG. 5, specific examples of the thoracoscopic surgery are as follows:
(1) Pneumonectomy, lung lobectomy, total pneumonectomy;
(2) Bra (spontaneous pneumothorax) resection;
(3) Mediastinal tumor resection;
(4) Cardiac bypass surgery; and
(5) Esophageal tumor resection surgery.

Specific examples of the laparoscopic surgery are as follows:
(6) Esophageal hiatal hernia surgery;
(7) Partial gastrectomy, subtotal gastrectomy, total gastrectomy;
(8) Gastric/duodenal ulcer perforation and plication;
(9) Gallbladder resection surgery;
(10) Liver cyst incision;
(11) Spleen resection surgery;
(12) Intestinal canal synechiotomy;
(13) Small bowel resection;
(14) Appendectomy;
(15) Colectomy;
(16) Inguinal hernioplasty;
(17) Adrenal resection surgery;
(18) Kidney resection surgery;
(19) Rectal resection; and
(20) Uterine fibroid nuclear surgery, partial oophorectomy.

Thus, the endoscope 100 can be applied to various surgical procedures, but the site where the endoscope 100 is inserted is different and the insertion direction is also different depending on each surgical procedure. For example, in the case of a surgical procedure to resect a lung, the endoscope 100 is inserted from an anterior axillary region B1 as illustrated in FIG. 6. When liver cancer is resected, the lens barrel 150 of the endoscope 100 is inserted from a navel B21 as illustrated in FIG. 7. In FIG. 7, three forcipes 70 are inserted between the navel B21 and a liver B22.

Even when the affected area is the same, the insertion location may differ depending on the surgical procedure. For example, when operating a kidney with a transperitoneal approach, as illustrated in FIG. 8(a), the lens barrel 150 of the endoscope 100 is inserted from a ventral side B31 of the flank toward the kidney, the forceps 70 on the right side is inserted from a middle B32 of the flank, and the forceps 70 on the left side is inserted from a hypochondriac region B33. When operating the kidney with the retroperitoneal approach, as illustrated in FIG. 8(b), the lens barrel 150 of the endoscope 100 is inserted from a middle B41 of the flank toward the kidney, the forceps 70 on the right side is inserted from the vicinity of a vertebral region B42 of the lumbar triangle, and the forceps 70 on the left side is inserted from a hypochondriac region B43.

In the surgical system 1 according to the present embodiment, when the information regarding the affected area and the surgical procedure is input from the input device 31, the initial position of the tip of the endoscope 100 is set in accordance with the input information, and the gripping robot 10 is appropriately moved/operated to adjust the position and insertion direction of the endoscope 100. The surgeon can therefore quickly and reliably insert the endoscope 100 into the patient.

The application to an inguinal hernia surgery will then be described as a specific example of surgery using the surgical system 1 according to the present embodiment.

In the case of inguinal hernia (surgical procedure information), the endoscope 100 is usually inserted from the navel (surgical procedure information) toward the inguinal region (organ information). When Dr. A who is the surgeon prefers to once insert the endoscope outside the inguinal region and then move the field of view inward (surgeon information), the initial position of the endoscope 100 is set so as to head to the outer position of the inguinal region from the navel.

Provided that the tip of the lens barrel 150 (rigid scope) of the endoscope 100 is located 10 cm away from the navel based on the endoscope product information and the adapter information (e.g., the tip of the rigid scope is located about 30 cm ahead of the adapter 11 at the tip of the gripping robot 10), the gripping robot 10 is moved so that if the tip of the rigid scope moves straight ahead without any change, it reaches the outside of the inguinal region. Here, the position of the patient's navel can be roughly calculated based on the patient information as to what position the patient's navel is located with respect to the origin provided on the surgical bed. If the relative position between the surgical bed and the gripping robot 10 is measured by a sensor or the like, the position to which the gripping robot 10 should move can be obtained.

By using the surgical system 1 according to the present embodiment, it is possible to specify the initial state of the gripping robot 10 from the information on the inguinal region (organ information) in the inguinal hernia (surgical procedure information), the preference of Dr. A who is the surgeon (surgeon information), the positional relationship between the endoscope 100 and the gripping robot 10 (treatment tool product information and adapter information), the patient information, etc., and the endoscope 100 can be automatically set to an initial position suitable when Dr. A performs surgery of the inguinal hernia.

As described above, according to the present embodiment, it is possible to provide the surgical system 1 capable of optimally controlling the initial position of a treatment tool based on multifaceted information regarding surgery.

Although the present embodiment and its specific examples have been described above, the present invention is not limited to these examples. For example, the endoscope 100 has been described as an example of the treatment tool, but the present invention can also be applied to other treatment tools than the endoscope 100, such as the forceps 70, a cleaning device, a suction device, and an ultrasonic scalpel. Furthermore, the scope of the present invention encompasses those to which a person skilled in the art appropriately makes addition or removal of constitutional elements or design changes with respect to the previously-described embodiments or their specific examples and those in which features of the embodiments are appropriately combined, provided that they have the subject matters of the present invention.

### [Description of Reference Numerals]

- 1: Surgical system
- 10: Gripping robot
- 11: Adapter
- 15: Arm
- 20: Initial setting unit
- 21: Storage unit
- 22: Position specifying unit
- 23: Drive control unit
- 24: Condition setting unit
- 31: Input device
- 32: Electronic medical record information
- 50: Another setting condition
- 60: Trocar
- 70: Forceps
- 100: Endoscope
- 110: Head portion
- 120: Operation unit
- 150: Lens barrel
- P1: Position
- P2: Position

## Claims

1. A surgical system comprising:
a gripping robot that holds a treatment tool; and
an initial setting unit that sets an initial state including an initial position of the gripping robot,
the initial setting unit comprising:
a storage unit that stores relevance information pertaining to a relationship between condition information regarding surgery and the initial position of the gripping robot;
a position specifying unit that sets, based on the condition information input and the relevance information input from the storage unit, the initial position of the gripping robot; and
a drive control unit that generates, based on a signal input from the position specifying unit, a control signal for moving a position of the gripping robot to a certain position and outputs the control signal to the gripping robot.

2. The surgical system according to claim 1, wherein the position specifying unit generates new relevance information based on a surgery performed immediately before by a surgeon and stores the new relevance information in the storage unit in association with the surgeon.

3. The surgical system according to claim 1 or 2, wherein the condition information includes one or more selected from a group consisting of surgeon information, surgical procedure information, patient information, target organ information, treatment tool product information, and adapter information of an adapter that is connected to the gripping robot and directly holds the treatment tool.

4. The surgical system according to claim 3, wherein the surgical procedure information includes information regarding a port position of a trocar.

5. The surgical system according to claim 3 or 4, wherein the patient information includes body shape, age, and gender.

6. The surgical system according to any one of claims 3 to 5, wherein the treatment tool product information includes information regarding a shape of the treatment tool.

7. The surgical system according to any one of claims 1 to 6, wherein the condition information includes at least one of information input from an input device, information on an electronic medical record, and information that is preliminarily stored in the storage unit.

8. The surgical system according to any one of claims 1 to 7, wherein
the storage unit also stores a relationship between the condition information and another setting in an operating room as the relevance information, and
the initial setting unit has a condition setting unit that specifies another setting condition in the operating room based on the condition information input and the relevance information input from the storage unit.
